(19) Europäisches Patentamt
European Patent Office
Office européen des brevets

(11) **EP 2 929 864 B1**

(12) **EUROPÄISCHE PATENTSCHRIFT**

(45) Veröffentlichungstag und Bekanntmachung des Hinweises auf die Patenterteilung:
**26.10.2016  Patentblatt 2016/43**

(21) Anmeldenummer: **15162470.7**

(22) Anmeldetag: **02.04.2015**

(51) Int Cl.:
*A61G 13/10* (2006.01)    *F21V 33/00* (2006.01)
*F24F 3/16* (2006.01)    *F24F 13/078* (2006.01)
*F24F 13/08* (2006.01)    *F21W 131/205* (2006.01)
*A61B 90/30* (2016.01)

(54) **REINRAUMBELEUCHTUNGSVORRICHTUNG**

CLEANROOM ILLUMINATION DEVICE

DISPOSITIF D'ÉCLAIRAGE DE SALLE BLANCHE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **07.04.2014   DE 102014004921**

(43) Veröffentlichungstag der Anmeldung:
**14.10.2015   Patentblatt 2015/42**

(73) Patentinhaber: **Innovations-Transfer Uphoff GmbH & Co. KG**
**35039 Marburg an der Lahn (DE)**

(72) Erfinder: **Schmitt, Wolfgang**
**57518 Betzdorf (DE)**

(74) Vertreter: **von Bülow & Tamada**
**Rotbuchenstraße 6**
**81547 München (DE)**

(56) Entgegenhaltungen:
**DE-A1- 3 331 299    US-A1- 2013 182 417**

**Beschreibung**

Die Erfindung betrifft eine Reinraumbeleuchtungsvorrichtung gemäß dem Oberbegriff des Patentanspruches 1.

Eine solche Reinraumbeleuchtungsvorrichtung ist aus der US 2013 0182 417 A1 bekannt. Diese Vorrichtung hat eine Luftstromerzeugungseinrichtung, einen Laminarisator in Form von Löchern in einer Deckenverkleidung und mehrere Lichtquellen, wobei wirksame Austrittsflächen der Lichtquellen in geschlossenen zylindrischen Röhren angeordnet sind. Diese Röhren sind in Öffnungen des Laminarisators angeordnet und erstrecken sich vollständig durch den Laminarisator hindurch. Der Rand der Röhren wirkt als stromabwärtige Strömungsabrisskante für die durch den Laminarisator laufende Luftströmung.

Die DE 10 2011 114 993 A1 beschreibt eine Reinraum-Sicherheitswerkbank mit einem laminaren Luftstrom, der aus Löchern eines Lochblechs austritt. In oder auch auf diesem Lochblech sind diverse LEDs vorgesehen, die aufgrund ihrer kleinen Größe den Luftstrom nicht stören.

Die DE 20 2005 009 505 U1 zeigt ein Schwebstofffilter für Reinräume, bei dem unterhalb des Filters ein Laminarisator vorgesehen ist, der durch ein Leuchtengehäuse durchstoßen wird. Innerhalb dieses Gehäuses befinden sich Leuchtmittel.

Die US 2011/0146676 A1 beschreibt einen medizinischen Operationsraum mit einer Vielzahl von Leuchtkörpern in Ausnehmungen einer Raumdecke. Wobei die einzelnen Leuchten verstellbar sind. Die Raumdecke enthält einen Laminarisator zur Erzeugung eines laminaren Luftstromes in Richtung zum Raum hin.

Die JP 62073026 A beschreibt eine Beleuchtungsvorrichtung für einen Reinraum, dessen Decke Luftfilter aufweist, die als Laminarisator wirken, wobei am Laminarisator LEDs vorgesehen sind.

In der Reinraumtechnik, wie zum Beispiel in Operationssälen mit hohen Anforderungen an die Keimarmut, muss einerseits dafür gesorgt werden, dass im Arbeitsbereich (Schutzbereich) turbulenzarme Luftströmungen vorhanden sind und andererseits dieser Bereich gut beleuchtet ist. Zur Erzeugung einer turbulenzarmen Strömung werden üblicherweise Laminarisatoren eingesetzt, die beispielsweise aus der EP 2522924 A2 oder der DE 10 2008 019698 B3 bekannt sind. Die Beleuchtung wird üblicherweise durch Deckenlampen realisiert, die seitlich neben den Laminarisatoren angeordnet sind sowie durch OP-Leuchten, die über eine Kardanik (Schwenkgestelle) aufgehängt sind und je nach Beleuchtungsanforderung positioniert und verschwenkt werden können.

Eine Beleuchtung allein durch Deckenlampen ist in der Praxis ungenügend hinsichtlich der Lichtstärke im Arbeitsbereich. OP-Leuchten mit Kardanik haben den Nachteil, dass die laminare Luftströmung gestört wird und daran Turbulenzen auftreten. Diese Turbulenzen können den Transport von Partikeln und auch Mikroorganismen sowohl vom Operateur (Gesicht, Kleidung etc.) in den Schutzbereich als auch vom Arbeitsbereich in den Atembereich des Operateurs bedingen oder begünstigen.

Aus der Reinraumtechnik ist die laminare (LAF) bzw. turbulenzarme Verdrängungsströmung (TAV) hinlänglich bekannt und hat Eingang in die allgemein anerkannten Regeln der Technik gefunden (DIN 1946 Teil 4, Raumlufttechnische Anlagen in Krankenhäusern, (2008) Beuth Verlag; DIN EN 13779, Lüftung von Nichtwohngebäuden, (2007) Beuth Verlag GmbH, DIN EN 13182. Lüftung von Gebäuden. Gerätetechnische Anforderungen für Messungen der Luftgeschwindigkeit in belüfteten Räumen (Dezember 2002) Beuth Verlag; VDI 2083 Reinraumtechnik, Blatt 2 (2005), Messtechnik in der Reinraumluft und Blatt 5 (1996), Thermische Behaglichkeit, Bau, Betrieb und Instandhaltung. Beuth Verlag).

Dabei ist der Turbulenzgrad als Maß für die Schwankungen bezogen auf den Mittelwert der Luftgeschwindigkeit entsprechend DIN EN 13182 bzw. VDI 2083 definiert. Er ist annähernd gleich der relativen Standardabweichung (Standardabweichung durch Mittelwert), wenn die Anzahl der Stichproben groß ist und die Richtung der Geschwindigkeitszeitschwankungen unberücksichtigt bleibt. Dabei werden drei Kenngrößen für den Turbulenzgrad unterschieden (VDI 2083 Teil 5, Tabelle 1): Strömung mit einem Turbulenzgrad von

```
< 5 %        □    laminar (LAF),

5-20 %   □    turbulenzarm (TAV),

> 20 %   □    turbulent.
```

LAF- bzw. TAV-Systeme finden überall dort Anwendung, wo aerogen übertragene partikuläre oder gasförmige Kontaminationen von Produkten und Arbeitsflächen, wie z. B. in industriellen Operationsräumen, vom Produkt (Produktschutz) oder den arbeitenden Personal (Personalschutz) ferngehalten werden muss.

Zur Herstellung eines Schutzbereichs mittels LAF- oder TAV-Strömung wird Zuluft zunächst durch Ventilatoren erzeugt, ggf. mittels HEPA-Filtern (High Efficiency Particulate Airfilter) steril filtriert und nach Durchströmen von "Laminarisatoren" gezielt in ganze Räume oder einen begrenzten Schutz- bzw. Arbeitsbereich eingebracht. Laminarisatoren sind im Stand der Technik bekannt, wurden in den letzten Jahrzehnten in vielen Varianten optimiert (EP 0355 517 B1) und erreichen eine Minderung des Turbulenzgrades, so dass von den überströmten Oberflächen weniger Energie mit der strömenden Luft ausgetauscht bzw. weniger Partikel entfernt oder deponiert werden.

Nach turbulenzarmer Durch- oder Überströmung des Schutzbereichs wird die Luft über Abluftöffnungen im Boden, an Wänden oder über Decken-fixierte Absaugungen abgeführt.

Typische Anwendungsbereiche für diese Technologie sind beispielsweise die Mikroelektronik zur Herstellung von EDV-Chips, die optische Industrie, Herstellungs- und Abfüllbereiche in der Pharmazie, Lebensmitteltechnik und die Operationsräume in der Medizin.

Speziell Operationsräume werden zur Erfüllung ihrer Aufgabenstellung als "Reinräume" entsprechend der DIN 1946 Teil 4, als allgemein anerkannter Regel der Technik, in solche der Raumklasse 1a (turbulenzarme Verdrängungsströmung) mit höchster raumlufttechnischer Reinheit sowie der Raumklasse 1b (turbulente Luftführung) mit geringerer raumlufttechnischer Reinheit unterschieden. Die Raumklasse 1a weist üblicherweise unter der OP-Raumdecke die Installation von mehreren (HEPA-) Zuluftfiltern auf. Über diese Filter und den anschließenden Laminarisator wird die minimal gegenüber der Raumluft kältere Zuluft zu dem vertikal darunter gelegenen Schutz- bzw. Arbeitsbereich aus Operationsfeld und Instrumententischen geführt. Üblicherweise und entsprechend der DIN 1946 Teil 4 befindet sich der Zuluft-Deckenauslass im mittleren Bereich des Operationsraumes und beansprucht eine Fläche von 3,2 x 3,2 Quadratmetern. Damit kann im Operationsraum ein lüftungstechnischer definierter Schutzbereich von 3 x 3 Quadratmetern abgebildet werden.

Um die Zuluft aus dem Deckenauslass für das OP-Team physiologisch-behaglich abströmen zu lassen, werden unterhalb der HEPA-Zuluftfilter "Laminarisatoren" (auch Gewebeverteiler, CG-Verteiler [CibaGeygi: erstmalige Anwendung]) als feinmaschiges (Kunststoff-)Gewebe gespannt. Diese Laminarisatoren erzeugen eine Strömung mit niedrigen Turbulenzgraden und somit einer gewünschten, gleichgerichteten, turbulenzarmen bzw. laminaren Luftströmung. In geeigneter Kombinationen aus Fadenstärke und Maschenweite lassen sich so laminare Strömungen (Turbulenzgrad: < 5 %) bis zur Arbeitsebene des Operationstisches (1-1,2 m OKFFB [Oberkante Fertigfußboden]) nachweisen.

Strömungshindernisse, die stromabwärts unterhalb der Ebene des Laminarisators liegen, erhöhen in der Regel den Turbulenzgrad bzw. wirken sich negativ auf die Qualität der Luftströmung im zu schützenden Arbeitsbereich aus. So ist beispielsweise von OP-Leuchten, deren Stativrohre durch die Ebene der Laminarisatoren geführt werden müssen, bekannt, dass sowohl die Stativdurchführung als auch die Leuchte den Turbulenzgrad lokal erhöhen. Weitere Beispiele für derartige Strömungsstörungen im Operationsraum im Bereich zwischen Laminarisator und Schutzbereich sind Deckenversorgungseinheiten (Medizinische Gase, Strom) und Bildschirme zur Prozessüberwachung.

Grundsätzlich zeichnen sich Reinräume neben diesen Luftführungs-systemen sowie aufgabenspezifischen (Bearbeitungs-) Geräten auch durch spezielle Beleuchtungssysteme aus. So werden z. B. in Reinräumen für medizinische Operationen und invasive Eingriffe an Menschen und Tieren speziell kombinierte Beleuchtungssysteme installiert. Dabei unterscheidet man nach DIN EN 12464-1 den "Bereich der Sehaufgabe", mit den höchsten Anforderungen an die Leuchtdichte, vom unmittelbaren "Umgebungsbereich" (ca. 0,5 m um das Gesichtfeld der Sehaufgabe) sowie den "Hintergrund" (ca. 3 m um die Sehaufgabe).

Für den "Bereich der Sehaufgabe" werden Operationsleuchten eingesetzt, die Leuchtdichten von 10- bzw. 40-160 kLx (DIN EN 12464-1, DIN EN 60601-2-4) in 1 m Abstand erreichen, so dass auch bei Patienten mit erheblicher Körpermasse noch die tief im Körper liegenden filigranen Gewebe und Organstrukturen möglichst fehlerfrei unterschieden, erkannt und behandelt werden können. Um beeinträchtigende Verschattungen, z. B. durch die Köpfe der Operateure, im Bereich der Sehaufgabe des Operationsfeldes zu minimieren, werden üblicherweise mindestens zwei Operationsleuchten redundant installiert und so eine ausreichende Beleuchtung sichergestellt.

Entsprechend DIN 5035-3 ist der "Umgebungsbereich" mit Leuchtdichten von 1,5-2 kLx sowie den "Hintergrundbereich" mit 1,0 kLx (DIN EN 12464-1) zu versorgen. Üblicherweise erfolgt die Ausleuchtung des "Umgebungsbereichs" mittels einer oder mehrerer nebeneinander angeordneten Reihen von Neo-Raumleuchten (2-3 reihig), welche umlaufend um den Zuluft-Deckenauslass an der OP-Raumdecke installiert sind. Diese ermöglichen dem OP-Team einerseits einen ausreichenden Überblick über die eingesetzten Instrumente und Geräte (z. B. für die Instrumentierende und die Anästhesie). Andererseits verhindert die Beleuchtung im "Umgebungs-" und "Hintergrundbereich", das das Operationsteam durch zu hohe Leuchtdichten-Unterschiede zum Bereich der Sehaufgabe Adaptationsstörungen ausgesetzt wird. Damit werden physiologisch akzeptable Kontraste sichergestellt und die Schutzbedingung entsprechend den gültigen Unfallverhütungsvorschriften (GUV-I-8681) erfüllt.

Als überwiegend übliche Installation werden seitlich um den Zuluft-Deckenauslass herum, an der OP-Raumdecke, mehr-reihige Neon-Leuchten angebracht. Nachteilig bei dieser seitlichen Anordnung ist, dass diese Leuchten in Richtung des OP-Bodens gerichtet sind und zum Schutzbereich, der mit einer Leuchtdichte von 1-2 kLx beleuchtet werden soll, aufgrund der Diagonale ein großer Abstand besteht.

Jedoch sind im Stand der Technik auch Beleuchtungssysteme bekannt, wobei Neon-Deckenleuchten zwischen den (HEPA-) Zuluftfiltern und dem darunter installierten Laminarisator angebracht sind. Vorteilhaft dabei ist, dass das emittierte Licht direkt von oben und damit auf kürzerem Weg auf den darunter liegenden Schutzbereich strahlt. Nachteilig an dieser Installationsart ist jedoch, dass die Lichtintensität durch den Laminarisator ganz erheblich gemindert wird.

Weiterhin nachteilig ist der erhöhte technische Aufwand zum Wechsel der Leuchtmittel, da hierzu der Laminarisator vollständig demontiert werden muss.

Im Verlauf einer Operation, welche durchschnittlich eine Stunde dauert (ca. 10 Minuten bis > 15 Stunden), ergeben sich laufend Änderungen der örtlichen Lage oder auch der Tiefe des vom OP-Team bearbeiteten Operationsfeldes. Um nach diesen prozessbedingten Lageänderungen der Sehaufgabe die erforderliche Ausleuchtung anzupassen, müssen die Operationsleuchten mittels der Kardanik jeweils neu positioniert werden. Diese Änderungen erfolgen üblicherweise manuell durch das OP-Team selbst oder durch Hilfskräfte ("Springer"), welche zur Erfüllung dieser Aufgabe speziell in den OP-Raum laufen müssen. Dabei stellt sich die korrekte Einrichtung der OP-Leuchten aufgrund der schwergängigen Kardanik als körperlich belastend und der erforderlichen sterilen Rahmenbedinungen auch als schwierig dar.

Aufgabe der vorliegenden Erfindung ist daher, eine Reinraumbeleuchtungsvorrichtung zu schaffen, mit der bei hoher Leuchtdichte eine turbulenzarme Strömung ausgebildet ist.

Diese Aufgabe wird durch die im Patentanspruch 1 angegebenen Merkmale gelöst. Vorteilhafte Ausgestaltungen und Weiterbildungen der Erfindung sind den Unteransprüchen zu entnehmen.

Die Grundidee der Erfindung besteht darin, die wirksame Austrittsfläche von Lichtquellen so in einen Laminarisator zu integrieren, dass sich eine kurze stromabwärtige Strecke nach den Lichtquellen wieder eine turbulenzarme Strömung ergibt. Konkret erreicht man dies dadurch, dass die wirksame Austrittsfläche von Lichtquellen in Gehäusen von rohrförmigen dünnwandigen Durchgangselementen angeordnet ist, die mit einem offenen Teil des Rohres über die Ebene des Laminarisators in den Raum hineinragen, so dass deren Rohrwände als scharfkantige Strömungsabrisskante wirken. Die Strömungsabrisskante erhält man also dadurch, dass die Gehäuse der Durchgangselemente die Form eines dünnwandigen Rohres haben, dessen zur Arbeitsfläche hin weisende Kante über die Fläche des Laminarisator hervorsteht. Dünnwandig bedeutet hier eine Wandstärke von kleiner gleich 5 mm und vorzugsweise kleiner gleich ca. 1,5 mm, wodurch erreicht wird, das sich eine kurze stromabwärtige Strecke nach der Strömungsabrisskante im wesentlichen keine Turbulenzen mehr bilden und damit dort im weiteren Strömungsverlauf bis zur Arbeitebene ein turbulenzarme Strömung vorhanden ist.

Die Lichtquelle kann beispielsweise eine herkömmliche Lampe oder eine LED-Beleuchtung sein, die unmittelbar in den Durchgangselementen angeordnet sind. Es ist aber auch möglich, die eigentliche Lichtquelle außerhalb der Durchgangselemente an zu ordnen und das Licht über Lichtleitfasern zu den Durchgangselementen zu führen, wobei als wirksame Lichtaustrittsfläche dann die Austrittsfläche der Lichtleitfasern verstanden wird.

Hierzu erfolgten Untersuchungen in einem Operationsraum mit einem TAV-Zuluftdeckenauslass von 3,2 x 3,2 qm (entsprechend Raumklasse 1a / DIN 1946 Teil 4) mit einem in 2,7 m Höhe OKFFB installierten Laminarisator. In 81 Messpositionen senkrecht unterhalb des Laminarisators (1 m Höhe OKFFB) wurden Turbulenzgradsonden installiert. Die 9 x 9 Messpositionen wiesen zueinander einen Abstand von 5 cm auf. Für Zuluftgeschwindigkeiten von 0,23 m/s, 0,27 m/s und 0,30 m/s fand sich auch auf der Arbeitsebene ein mittlerer Turbulenzgrad von < 5 % (laminare Strömung).

Anschließend wurden auf der Abströmseite des Laminarisators nacheinander Metallscheiben von 80, 120, 160 und 230 mm Durchmesser (0,4 mm Stärke) flächendeckend fixiert. Dann erfolgte in der jeweiligen Konstellation die Bestimmung der Turbulenzgrade im Vergleich zu den Ausgangsbedingungen (ohne Metallscheibe). Als Ergebnis zeigte sich bei allen Zuluftgeschwindigkeiten, dass die Metallscheiben bis 120 mm Durchmesser auf der Arbeitsebene keine Überschreitungen des Turbulenzgrades von 5 % bewirkten. Bei dem Metallscheiben-Durchmesser von 160 mm wurde der Turbulenzgrad von 5 % bei allen Zuluftgeschwindigkeiten überschritten.

Es folgten Visualisierungsuntersuchungen, indem in Strömungsrichtung unterhalb der Metallröhren Aerosolnebel über Kunststoffsonden injiziert wurde. Die turbulente Strömung unterhalb der Metallscheiben wurde dabei in Abhängigkeit von der Strömungsgeschwindigkeit photographiert und ausgemessen. Als Ergebnis zeigte sich, dass die turbulente Strömung unterhalb der Metallplatte grundsätzlich eine halbkegelförmige Form annahm, deren Länge in keinem Fall den 1,2-fachen Durchmesser der Metallscheibe überschritt.

Mit dem gleichen Versuchsaufbau wie für runde Metallscheiben wurden weitere Untersuchungen mit verschiedenen Formen durchgeführt. Dabei konnte zusammenfassend ermittelt werden, dass neben runden und ovalen auch (vier- und sechs-) eckige Formen zu keiner Überschreitung des Turbulenzgrades von 5 % führten, wenn diese einen Durchmesser < 160 mm aufwiesen.

Insbesondere jedoch stellte sich heraus, dass der Turbulenzgrad in der Arbeitsebene bei dem Durchmesser von 160 mm dadurch erheblich reduziert werden konnte, wenn anstatt der Metallplatten Metallröhren verwendet wurden, die (analog wie die Metallplatten) geschlossen auf dem Laminarisator fixiert waren, aber eine "scharfe Abrisskante" aufwiesen, die mindestens 1 mm über die stromabwärtige Seite des Laminarisators hervorstanden.

Die Erfindung basiert daher primär auf der Erkenntnis, dass ein in den Laminarisator eingebrachtes Durchgangselement (LPE für Laminarisator-Passage-Element) zunächst lokal stromabwärts eine halbkugel-förmige turbulente Strömung erzeugt. Durch geeignete Auswahl von Form und Durchmesser des Durchgangselementes (LPE) kann jedoch aufgrund der seitlich abströmenden turbulenzarmen Luft nach etwa dem maximalen Durchmesser des LPE wieder eine turbulenzarme Strömungsform erreicht werden. Dabei wird die Arbeitsebene im Schutzbereich wieder mit turbulenzarmer Strömung beaufschlagt.

Aufgrund dieser Erkenntnisse ergibt sich als Applikation die Integration von Lampen in den Laminarisator.

Durch Verzicht auf die klassischen -OP-Leuchten mit Kardanik kann die Ebene des Laminarisators von (derzeit üblich: 2,7-3 m) auf ≤ 2,5 m abgesenkt werden. Durch diese Reduktion zwischen Laminarisator- und Arbeitsebene wird die Leuchtdichte auf der Arbeitsebene wesentlich erhöht.

Im Folgenden wird die Erfindung anhand von Ausführungsbeispielen im Zusammenhang mit der Zeichnung ausführlicher erläutert. Es zeigt:

Fig. 1  eine schematische Schnittansicht eines Reinraumes mit der Beleuchtungsvorrichtung nach der Erfindung;

Fig. 2  eine Ansicht ähnlich Fig. 1 mit Darstellung von Lichtstrahlen;

Fig. 3  eine Ansicht ähnlich Fig. 2 mit auf einen engeren Arbeitsbereich konzentrierten Lichtstrahlen;

Fig. 4  eine schematische Draufsicht auf die Reinraumbeleuchtungsvorrichtung nach der Erfindung.

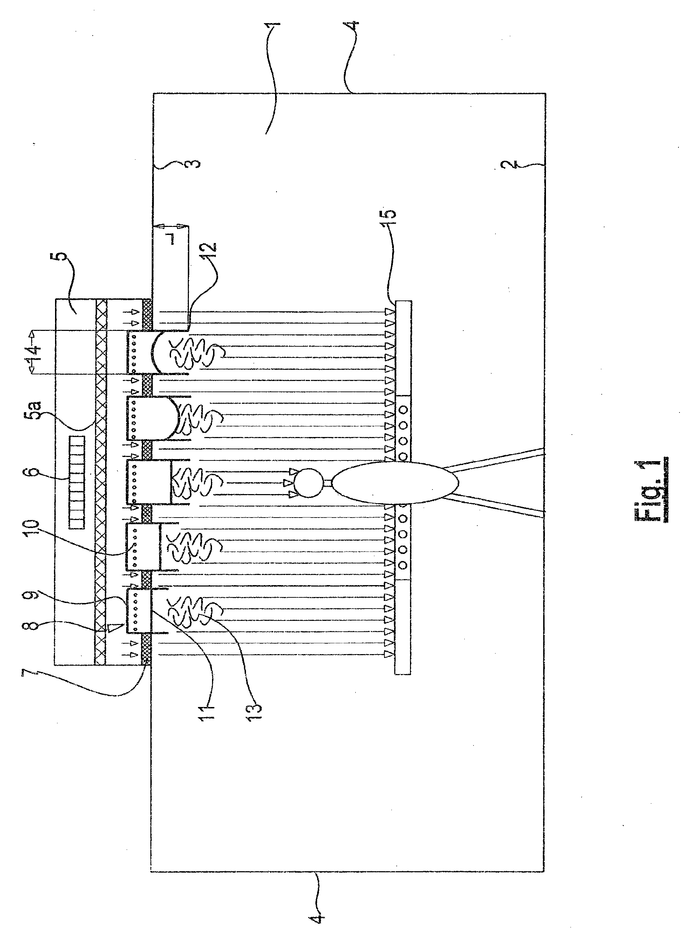

Fig. 1 zeigt einen Reinraum 1 mit einem Boden 2, einer Raumdecke 3 und Seitenwänden 4. An der Raumdecke 3 ist eine Luftzufuhreinrichtung 5 angebracht, die einen Ventilator 6, einen Filter 5 a, und einen Laminarisator 7 aufweist. Der Laminarisator 7 hat eine Vielzahl von Öffnungen, in die Beleuchtungseinrichtungen 8 in Form von Durchgangselementen eingesetzt sind. Die Beleuchtungseinrichtungen 8 haben ein geschlossenes Gehäuse 9, in dem Leuchtmittel bzw. Lichtquellen 10 angeordnet sind. Die Gehäuse haben an der in Richtung zum Inneren des Reinraumes 1 weisenden stromabwärtigen Seite eine lichtdurchlässige Abdeckung 11, die eben sein kann, aber auch konvex oder konkav gewölbt, um das Licht zu bündeln oder zu streuen. Die Gehäuse 9 ragen über die Unterseite des Laminarisators 7 in den Reinraum 1 hinein und haben eine scharfe Strömungsabrisskante 12. Die Gehäuse 9 haben die Form eines dünnwandigen Rohres, das stirnseitig geschlossen ist. Die stromabwärtige Kante des Gehäuses 9, die als Strömungsabrisskante 12 bezeichnet wird, ragt mindestens 1 mm (Maß L in Fig. 1) aus der Ebene des Laminarisators 7 heraus. Bei einem Ausführungsbeispiel der Erfindung entspricht diese Ebene der Decke 3 des Reinraumes 1.

Die Querschnittsform der Gehäuse 9 ist weitestgehend frei wählbar und kann z. B. quadratisch, kreisförmig, elliptisch oder n-eckig sein mit n > 3. Die Wandstärke des Gehäuses beträgt an der Strömungsabrisskante 12 bis maximal 3 mm und vorzugsweise weniger als 1,5 mm.

Die von dem Ventilator 6 geförderten Luftströmungen laufen durch den Laminarisator 7 hindurch und umströmen die Gehäuse 9. In Strömungsrichtung unterhalb der Gehäuse 9 bilden sich Turbulenzen, was durch Linien 13 angedeutet sind. Aufgrund der Strömungsabrisskante 12 an den Gehäusen 9 hat der Bereich der Turbulenz eine etwa halbkugelförmige Form mit einer Länge, die in etwa der Breite 14 der Gehäuse 9 entspricht und nicht größer ist, als etwa das 1,2 fache des größten Durchmessers der Gehäuse 9. Nach diesem Bereich ist die Strömung wieder, den Anforderungen der jeweiligen Reinraumqualität entsprechend, laminar oder turbulenzarm.

Bei ausreichendem Abstand zwischen dem in den Raum 1 ragenden Gehäuse 9 und einer Arbeitsebene 15 ist somit sichergestellt, dass laminare oder turbulenzarme Strömung an der Arbeitsebene 15 ankommt. Gleichzeitig kann durch die Lichtquellen 10, deren Leuchtmittel vorzugsweise Glasfaser oder LEDs sind, eine Beleuchtung der Arbeitebene 15 mit wesentlich erhöhten Leuchtdichten erreicht werden.

Die Lichtquellen 10, die auch die wirksamen Lichtaustrittsflächen von Glasfaserleitungen sein können, sind innerhalb der Gehäuse 9 schwenkbar, so dass gezielt bestimmte Bereiche der Arbeitsebene 15 beleuchtet werden können. Das Schwenken kann auch durch Spiegel erfolgen. Das Schwenken erfolgt weiter über ferngesteuerte Antriebe. Auch können die Lichtquellen 10 innerhalb der Gehäuse 9 fokussiert werden, was beispielsweise durch ebenfalls vorzugsweise ferngesteuerte verstellbare Linsensysteme der lichtdurchlässigen Abdeckung 11 erreicht wird.

Die Gehäuse 9 werden deshalb als Durchgangselemente bezeichnet, weil sie sich vollständig durch den Laminarisator 7 hindurch erstrecken, also durch ihn hindurchgehen.

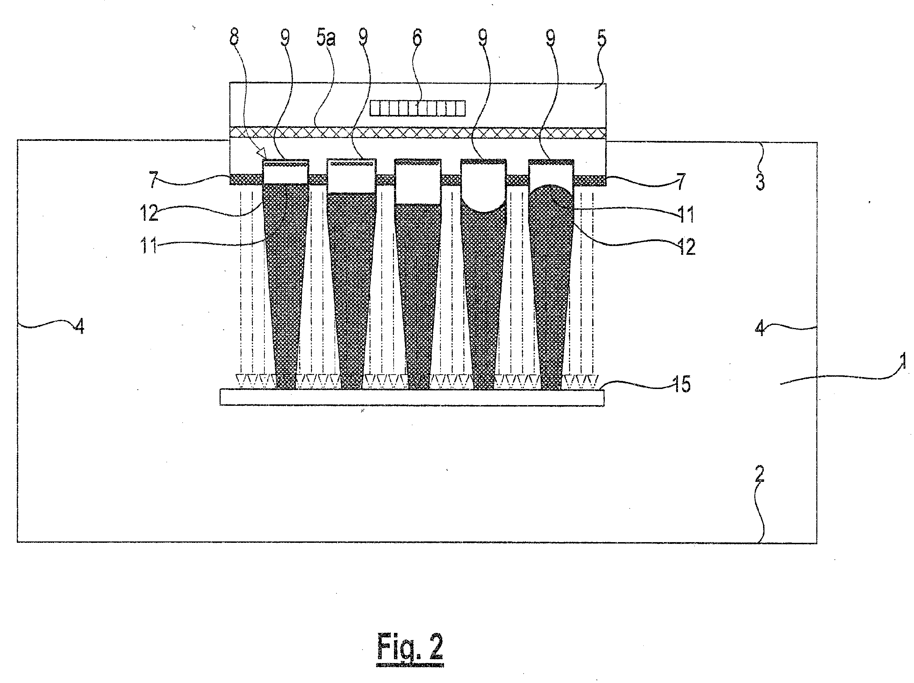

Ein weiterer Vorteil der Erfindung ist in Fig. 2 dargestellt. Durch den Verzicht auf die klassischen OP-Leuchten mit Kardanik kann die Ebene des Laminarisators 7 von derzeit üblichen 2,7-3 m auf ≤ 2,5 m abgesenkt werden. Durch diese Reduktion zwischen der Ebene des Laminarisators 7 und der Arbeitsebene 15 wird die Leuchtdichte an der Arbeitsebene 15 wesentlich erhöht, da die Leuchtdichte bekanntlich mit dem Quadrat der Entfernung zwischen Lichtquelle und bestrahltem Objekt abnimmt.

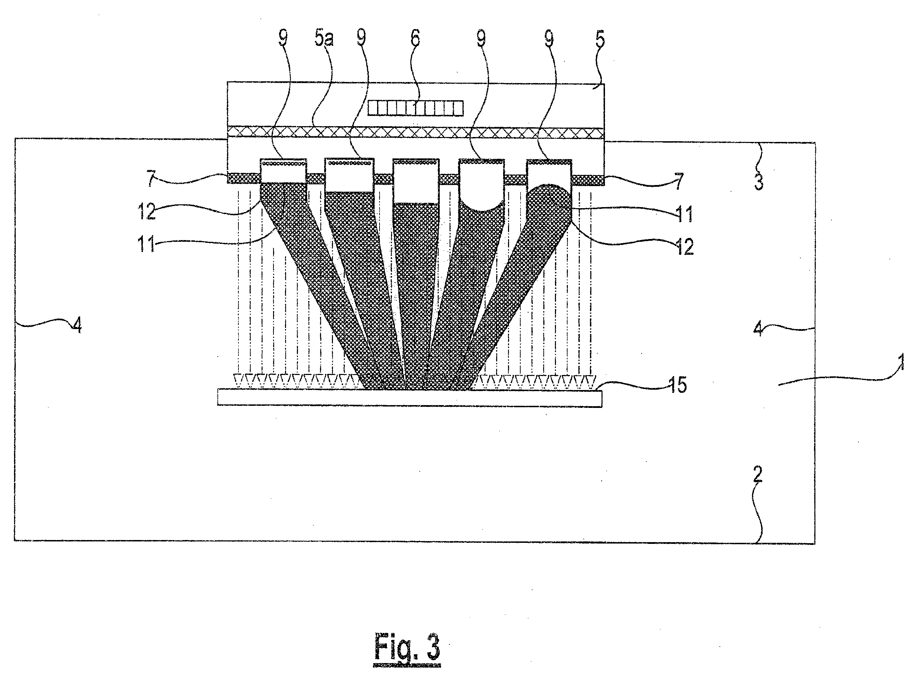

Weiter entfällt das eingangs genannte Hantieren mit der Kardanik der OP-Leuchten, da die einzelnen Lichtquellen im LPE ferngesteuert schwenkbar angeordnet sind, damit das Licht mehrerer Quellen auf einen engeren Arbeitsbereich konzentriert und so auch die lokale Fokussierung verändert werden kann, was in Fig. 3 dargestellt ist.

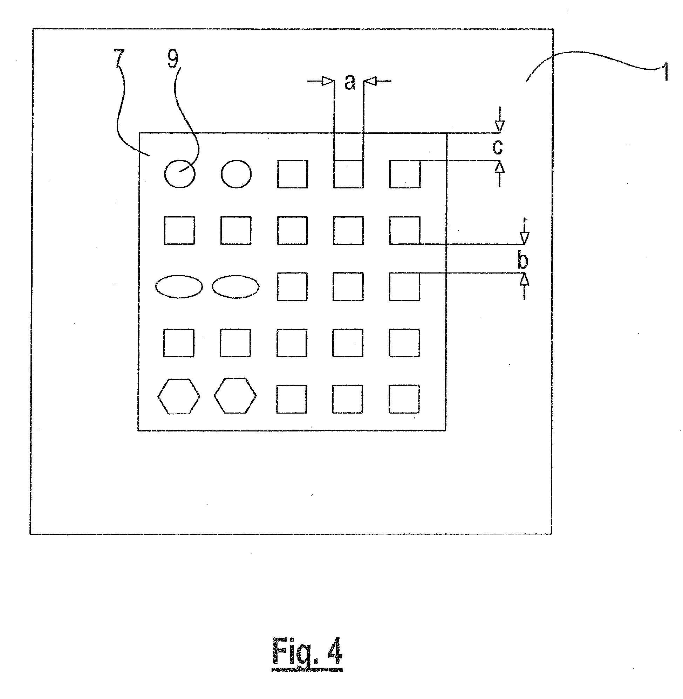

Fig. 4 zeigt eine Draufsicht auf den Laminarisator 7 mit mehreren eingesetzten Gehäusen 9 und verdeutlicht, dass die Gehäuse 9 sehr unterschiedliche Formen haben können, wie z. B. quadratisch, kreisförmig, elliptisch, n-eckig, mit n > 3, wie z.B. sechseckig usw.

In einem konkreten Ausführungsbeispiel mit einem Laminarisator 7 einer gesamten Fläche von 3,2 m x 3,2 m in einer Höhe von 2,7 m betrug der Durchmesser a der Gehäuse 12 cm. Der Abstand b zwischen zwei benachbarten Gehäusen 9 betrug 5 cm und der Abstand c der Gehäuse 9 zum Rand des Laminarisators 7 betrug 10 cm. Mit diesen Abmessungen wurde unterhalb der Gehäuse 9 nach einem Abstand, der maximal das 1,2 fache des Durchmessers a der Gehäuse 9 betrug, visuell ein Zusammenschluss der Strömung dokumentiert und messtechnisch in der Arbeitsebene stets ein Turbulenzgrad < 5 % ermittelt.

Die oben angegebenen Abmessungen haben sich in dem konkreten Ausführungsbeispiel als günstig erwiesen. Dem Fachmann ist klar, dass auch andere Abmessungen verwendet werden können, sofern sichergestellt ist, dass in ausreichendem Abstand oberhalb der Arbeitsebene 15 eine turbulenzarme oder laminare Strömung vorhanden ist.

## Patentansprüche

1. Reinraumbeleuchtungsvorrichtung mit einer Luftstromerzeugungseinrichtung (6), einem Laminarisator (7) und mit mehreren Lichtquellen (10),
   wobei wirksame Austrittsflächen der Lichtquellen (10) in geschlossenen Gehäusen (9) angeordnet sind,
   die Gehäuse (9) in Öffnungen des Laminarisators (7) angeordnet sind und sich die Gehäuse (9) als Durchgangselemente vollständig durch den Laminarisator (7) hindurch erstrecken und die Gehäuse (9) stromabwärtige Strömungsabrisskanten (12) für die durch den Laminarisator (7) laufende Luftströmumg aufweisen, **dadurch gekennzeichnet,**
   **dass** die Strömungsabrisskanten (12) dadurch gebildet sind, dass die Durchgangselemente (9) dünnwandige Rohre sind, deren stromabwärtige Strömungsabrisskante (12) über die Ebene des Laminarisators (7) vorsteht,
   wobei sich die dünnwandigen Rohre an ihren in Richtung des Raumes zeigenden Stirnseiten über eine mögliche lichtdurchlässige Abdeckung (11) hinaus erstrecken, so dass ein offener Teil der Rohre in den Raum hineinragt, deren Rohrwände als scharfkantige Stromabrisskanten wirken.

2. Reinraumbeleuchtungsvorrichtung nach Anspruch 1, **dadurch gekennzeichnet,**
   **dass** die Wanddicke der Durchgangselemente $\leq$ 3 mm und vorzugsweise $\leq$ 1,5 mm ist.

3. Reiriraumbeleuchtungsvorrichtung nach Anspruch 1 oder 2, **dadurch gekennzeichnet, dass** die stromabwärtige Strömungsabrisskante (12) der Durchgangselemente mindestens 1 mm (L) über die Ebene des Laminarisators (7) vorsteht.

4. Reinraumbeleuchtungsvorrichtung nach einem der Ansprüche 1-3, **dadurch gekennzeichnet, dass** die Lichtquellen (10) innerhalb der Durchgangselemente (9) schwenkbar sind.

5. Reinraumbeleuchtungsvorrichtung nach Anspruch 4, **dadurch gekennzeichnet,**
   **dass** das Licht der Lichtquellen (10) innerhalb der Durchgangselemente (9) durch Spiegel schwenkbar ist.

6. Reinraumbeleuchtungsvorrichtung nach einem der Ansprüche 1-5, **dadurch gekennzeichnet, dass** das Licht der Lichtquellen (10) innerhalb der Durchgangselemente (9) fokussierbar ist.

7. Reinraumbeleuchtungsvorrichtung nach einem der Ansprüche 1-6 , **dadurch gekennzeichnet, dass** die Durchgangselemente (9) auf ihrer zum Inneren eines Raumes (1) weisenden Seite eine lichtdurchlässige Abdeckung (11) aufweisen.

8. Reinraumbeleuchtungsvorrichtung nach Anspruch 7, **dadurch gekennzeichnet,**
   **dass** die lichtdurchlässige Abdeckung (11) eine Linse ist.

9. Reinraumbeleuchtungsvorrichtung nach einem der Ansprüche 1-8, **dadurch gekennzeichnet, dass** die Durchgangselemente (9) in Draufsicht quadratisch, kreisförmig, elliptisch oder n-eckig, mit n > 3, sind.

10. Reinraumbeleuchtungsvorrichtung nach einem der Ansprüche 1 bis 9, **dadurch gekennzeichnet, dass** die Lichtquellen (10) mindestens ein Glasfaser-Lichtkabel oder ein LED aufweisen.

**11.** Verwendung einer Reinraumbeleuchtungsvorrichtung nach einem der Ansprüche 1-8, **dadurch gekennzeichnet, dass** der Abstand zwischen der Strömungsabrisskante (12) der Durchgangselemente (9) und dem Boden (2) des Raumes (1) auf maximal 2,5 m eingestellt wird.

**Claims**

**1.** Device for illuminating clean rooms with an air stream generation mechanism (6), a laminarisator (7) and with several light sources (10),
wherein effective light exit fields of the light sources (10) are arranged in closed housings (9),
the housings (9) are arranged in openings of the laminarisator (7) and the housings (9) extend completely as passing elements through the laminarisator (7) and the housings (9) include downstream stall flanges (12) for the air stream passing the laminarisator (7), **characterized in**
**that** the stall flanges (12) are formed in that the passing elements (9) are thin walled tubes, which downstream stall flange (12) protrudes over the layer of the laminarisator (7),
wherein the thin walled tubes extend at its end faces oriented into the direction of the room beyond a possible light passing cover (11), such that an open part of the tubes, which tube walls effect as sharp edged stall flanges protrudes into the room.

**2.** Device for illuminating clean rooms according to claim 1, **characterized in**
**that** the wall thickness of the passing elements is ≤ 3 mm and preferably ≤ 1,5 mm.

**3.** Device for illuminating clean rooms according to claim 1 or 2, **characterized in**
**that** the downstream stall flange (12) of the passing elements protrudes at least 1 mm (L) beyond the layer of the laminarisator (7).

**4.** Device for illuminating clean rooms according to one of the claims 1 to 3, **characterized in**
the light sources (10) are rotatable within the passing elements (9).

**5.** Device for illuminating clean rooms according to claim 4, **characterized in**
**that** the light of the light sources (10) within the passing elements (9) is rotatable via mirrors.

**6.** Device for illuminating clean rooms according to one of the claims 1 to 5, **characterized in**
the light of the light sources (10) is focusable within the passing elements (9).

**7.** Device for illuminating clean rooms according to one of the claims 1 to 6, **characterized in**
**that** the passing elements (9) include at their face side oriented to the inner of the room (1) a light passing cover (11).

**8.** Device for illuminating clean rooms according to claim 7, **characterized in**
**that** the light passing cover (11) is a lense.

**9.** Device for illuminating clean rooms according to one of the claims 1 to 8, **characterized in**
**that** the light passing cover (9) has in a top view a quadratic, circular, elliptical or n-edge shape, wherein n > 3.

**10.** Device for illuminating clean rooms according to one of the claims 1 to 9, **characterized in**
**that** the light source (10) includes at least a glass fiber light cable or a LED.

**11.** Use of a device for illuminating clean rooms according to one of the claims 1 to 8, **characterized in that** the distance between the stall flange (12) of the passing elements (9) and the bottom (2) of the room (1) is set to a maximum of 2,5 m.

**Revendications**

**1.** Dispositif d'éclairage de salle blanche ayant un dispositif de génération de flux d'air (6), un dispositif de mise en forme laminaire (7) et une pluralité de sources de lumière (10),

dans lequel les surfaces efficaces de sortie des sources de lumière (10) sont disposées dans des boîtiers (9) fermés, les boîtiers (9) sont agencés dans des ouvertures du dispositif de mise en forme laminaire (7), et les boîtiers (9) comme éléments de passage traversent ioentièrement le dispositif de mise en forme laminaire (7), et les boîtiers (9) comprennent des bords de séparation d'écoulement (12) en aval pour le flux d'air s'écoulant à travers le dispositif de mise en forme laminaire (7), **caractérisé en ce que**

les bords de séparation d'écoulement (12) sont formés de façon que les éléments de passage (9) soient des tubes à paroi mince, la partie aval des bords de séparation d'écoulement (12) dépassant au-delà de la surface du dispositif de mise en forme laminaire (7),

dans lequel les tubes à paroi mince s'étendent selon leur face frontale en direction de l'espace au-delà d'un éventuel couvercle (11) translucide, de sorte qu'une partie ouverte des tubes fait saillie dans la salle et que les parois des tubes forment des arêtes effilées de séparation d'écoulement.

2. Dispositif d'éclairage de salle blanche selon la revendication 1, **caractérisé en ce que** l'épaisseur de paroi des éléments de passage est ≤ 3 mm et de préférence ≤ 1,5 mm.

3. Dispositif d'éclairage de salle blanche selon l'une des revendications 1 ou 2, **caractérisé en ce que** la partie aval des bords de séparation d'écoulement (12) des éléments de passage dépasse d'au moins 1 mm (L) au-delà de la surface du dispositif de mise en forme laminaire (7).

4. Dispositif d'éclairage de salle blanche selon l'une des revendications 1-3, **caractérisé en ce que** les sources de lumière (10) peuvent être pivotées à l'intérieur des éléments de passage (9).

5. Dispositif d'éclairage de salle blanche selon la revendication 4, **caractérisé en ce que** la lumière des sources de lumière (10) peut être pivotée au moyen d'un miroir à l'intérieur des éléments de passage (9).

6. Dispositif d'éclairage de salle blanche selon l'une quelconque des revendications 1-5, **caractérisé en ce que** la lumière des sources de lumière (10) peut être focalisée à l'intérieur des éléments de passage (9).

7. Dispositif d'éclairage de salle blanche selon l'une quelconque des revendications 1-6, **caractérisé en ce que** les éléments de passage (9) comportent un couvercle translucide (11) selon leur côté orienté vers l'intérieur d'une salle (1).

8. Dispositif d'éclairage de salle blanche selon la revendication 7, **caractérisé en ce que** le couvercle translucide (11) est une lentille.

9. Dispositif d'éclairage de salle blanche selon l'une quelconque des revendications 1-8, **caractérisé en ce que** les éléments de passage (9) ont en vue de dessus une forme carrée, circulaire, elliptique ou à n côtés avec n > 3.

10. Dispositif d'éclairage de salle blanche selon l'une quelconque des revendications 1 à 9, **caractérisé en ce que** les sources de lumière (10) comprennent au moins un guide de lumière à fibre de verre ou une LED.

11. Dispositif d'éclairage de salle blanche selon l'une quelconque des revendications 1-8, **caractérisé en ce que** la distance entre les bords de séparation d'écoulement (12) des éléments de passage (9) et le sol (2) de la salle (1) est ajustée à 2,5 m maximum.

**Fig.1**

Fig. 2

EP 2 929 864 B1

Fig. 3

Fig. 4

**EP 2 929 864 B1**

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 20130182417 A1
- DE 102011114993 A1
- DE 202005009505 U1
- US 20110146676 A1
- JP 62073026 A
- EP 2522924 A2
- DE 102008019698 B3
- EP 0355517 B1